(19) Europäisches Patentamt
European Patent Office
Office européen des brevets

(11) Publication number: 0 132 049
B1

# (12) EUROPEAN PATENT SPECIFICATION

(45) Date of publication of the patent specification: **29.10.86**

(51) Int. Cl.[4]: **C 07 C 51/47**, C 07 C 53/02, C 07 C 53/08, C 07 C 53/122, C 07 C 53/126

(21) Application number: **84304026.2**

(22) Date of filing: **14.06.84**

(54) **Process for separating a carboxylic acid of 1-8 carbon atoms from a mixture thereof with water and/or one or more other oxygenated aliphatic compounds.**

(30) Priority: **17.06.83 GB 8316540**

(43) Date of publication of application: **23.01.85 Bulletin 85/4**

(45) Publication of the grant of the patent: **29.10.86 Bulletin 86/44**

(84) Designated Contracting States: **DE FR GB IT**

(56) References cited:
**US - A - 4 329 280**
**US - A - 4 404 145**

(73) Proprietor: **The British Petroleum Company p.l.c., Britannic House Moor Lane, London EC2Y 9BU (GB)**

(72) Inventor: **Groszek, Alexander Jerzy, The British Petroleum Company p.l.c. Chertsey Road, Sunbury-on-Thames Middlesex, TW16 7LN (GB)**

(74) Representative: **Krishnan, Suryanarayana Kalyana et al, c/o The British Petroleum Company plc Patents Division Chertsey Road, Sunbury-on-Thames Middlesex TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

**Description**

The present invention relates to a process for separating a carboxylic acid from a mixture thereof with water and/or one or more other oxygenated aliphatic compounds.

In any process for separating components of a mixture by adsorption the adsorbent should possess the following properties:

1. A high capacity for preferential adsorption,
2. A rapid rate of adsorption (enabling the adsorbent to adsorb a significant amount of the desired component in a short time from a mixture with which it is contacted), and
3. A high selectivity for the desired component so that effective separation of the component from mixtures in which its concentration is low can be effected.

A class of adsorbents which possess these properties is the crystalline aluminosilicates, the best known of which are the zeolithes. The crystalline aluminosilicates function as «molecular sieves», that is they contain pores having cross-sectional diameters which will accept certain molecules in a mixture of molecules of specific size and shape while rejecting others. A particular type of synthetic crystalline aluminosilicate which has come to fore in recent years both as a catalyst and as an adsorbent is the MFI-type zeolithe, which is defined in the «Atlas of Zeolite Structure Types» by Meier et al, published by the Structure Commission of the Zeolite Association (1978). A typical MFI-type zeolite is that known as ZSM-5 which is described and claimed in US Patent No 3 702 886 (Mobil). A ZSM-5-type zeolite has the composition in terms of mole ratios of oxides:

$$0.9 \pm 0.2\ M_2/_nO : Al_2O_3 : 5\text{-}100\ SiO_2 : zH_2O$$

wherein M is a cation, n is the valency of the cation and z is from 0 to 40 and a characteristic X-ray diffraction pattern. Another type of material rapidly gaining acceptance as an adsorbent is that known as silicalite, which is a hydrophobic crystalline silica molecular sieve described and claimed in US Patent No 4 061 724 (Union Carbide). Silicalite is regarded by some (see, for example, Nature, Vol 300, 25.11.82, page 309, «Research article triggers dispute on zeolite» by Stephen Budiansky) as an extreme form of ZMS-5 in which the silicon to aluminium ratio is very high, for example about 500 : 1. In contrast to low silica to alumina molar ratio zeolites, due to its essentially aluminium free structure, silicalite does not show any detectable ion-exchange behaviour, and is both hydrophobic and organophilic. The separation process utilising silicalite contemplated in US Patent No 4 061 724 comprises, in general terms, the separation of an organic compound from an aqueous solution. The organic molecules specifically exemplified are n-butanol, methyl cellosolve, methanol and phenol.

In US Patent No 4 367 364 there is described a process for separating a normal paraffin from a mixture of the same with another structural class of hydrocarbon selected from the cyclic hydrocarbons having greater than six carbon atoms per molecule and the branched chain hydrocarbons, which comprises contacting said mixture at adsorption conditions with an adsorbent consisting essentially of silicalite to effect the selective adsorption of said normal aliphatic hydrocarbon by said adsorbent.

In our copending unpublished UK application No 8 222 621 (BP Case No 5405) there is described a process for the separation of ethanol from an aqueous solution thereof which comprises bringing the aqueous solution into contact with a zeolite which is a high silica zeolite wherein the molar ratio of silica to alumina is in the range 300 : 1 to 12 : 1.

We have now found that a carboxylic acid can be separated from a mixture thereof with water and/or one or more other oxygenated aliphatic compounds by contacting the mixture with either silicate or an MFI-type zeolite having a silica to alumina molar ratio greater than 50 : 1. This is surprising since there is no evidence to suggest that high silica MFI-type zeolites possess sites which would have an especially strong affinity for the acidic carboxylic group.

Accordingly, the present invention is a process for separating a carboxylic acid from a mixture thereof with water and/or one or more other oxygenated aliphatic compounds which process comprises contacting the mixture under adsorption conditions with an adsorbent comprising either silicalite or an MFI-type crystalline aluminosilicate zeolite having a silica to alumina molar ratio greater than 50 : 1.

As mentioned hereinbefore, the preparation and characterisation of silicate is described in US Patent No 4 061 724, which is incorporated herein by reference. Silicalite may be prepared for example by the hydrothermal crystallisation of a reaction mixture comprising water, a source of silica and an alkylonium compound, suitably a quaternary ammonium hydroxide or salt, at a pH of 10 to 14 to form a hydrous crystalline precursor, and subsequently calcining that precursor to decompose alkylonium moieties present therein. Silicalite is the preferred adsorbent for use in the process of the invention.

MFI-type crystalline aluminosilicate zeolites may suitably be prepared by crystallisation for a time greater than 0.5 hr at a temperature in the range from 80 to 220°C, preferably from 120 to 175°C, from a gel comprising (i) a source of silica ($SiO_2$), (ii) a source of alumina ($Al_2O_3$), (iii) a mineralising agent selected from oxides and salts of alkali and alkaline earth metals ($X_2/_bO$ wherein b is the valency of X), (v) an organic base (B), as hereinafter defined, and (vi) water and/ore alcohol ROH).

The term organic base as used hereinbefore means any organic material for which $-10_{10}$ K for the reaction:

$$B + H_{20} = BH^+ + OH^-$$

is less than 7, where $K = [BH^+][OH^-]/[B]$

Suitable organic bases include:

(iii) tetrahydrocarbylammonium salts of the formula:

$$R^1R^2R^3R^4N^+X^-$$

wherein $R^1$, $R^2$, $R^3$ and $R^4$ are independently

either aryl, substituted aryl, alkyl, substituted alkyl, cycloalkyl, substituted cycloalkyl or hydrogen and $X^-$ is an inorganic anion. Preferably $R^1$, $R^2$, $R^3$ and $R^4$ are alkyl groups containing from 1 to 5 carbon atoms. Preferably $X^-$ is a halide or hydroxide ion. Suitable tetraalkylammonium salts include tetraethylammonium hydroxide and tetrapropylammonium hydroxide. Alternatively, the precursors of tetraalkylammonium compounds may be used.

(ii) amines having the formula:

either $R^1R^2R^3N$, or

$R^1R^2N(CH_2)_xNR^3R^4$ wherein x is an integer in the range 1 to 20, or $R^1R^2N(CH_2)_yN(CH_2)_zNR^3R^4$ wherein y and z are integers in the range 1 to 20. Suitable amines include $C_1$ to $C_9$ primary monoalkyl amines, such as n-ethylamine, n-propylamine and n-butylamine.

(iii) Mono-, di- and tri-alkanolamines such as monoethanolamine, diethanolamine and triethanolamine, or their precursors in the form of an alkylene oxide and ammonia.

(iv) aqueous ammonia.

Suitable sources of silica include, for example, sodium silicate, silica hydrosol, silica gel, silica sol and silicic acid. The preferred source of silica is an aqueous colloidal dispersion of silica particles. A suitable commercially available source of silica is LUDOX* Colloidal Silica manufactured by DuPont (* Registered Trade Mark).

Suitable sources of alumina include, for example, sodium aluminate, aluminium sulphate and alumina. The preferred source of alumina is sodium aluminate prepared by dissolving particulate alumina in excess sodium hydroxide solution.

The preferred mineralising agents are alkali and alkaline earth metal hydroxides and halides, such as for example, lithium hydroxide, sodium hydroxide, potassium hydroxide, potassium bromide and calcium bromide. The preferred mineralising agent is sodium hydroxide.

In will be appreciated that each source of silica, alumina, alkali or alkaline earth metal can be supplied by one or more initial reactants and then mixed together in any order. For example, sodium silicate is a source of both alumina and silica and an aluminosilicate is a source of both alumina and silica. Thus, the source of alumina and the source of silica may be supplied in whole or in part by an aluminosilicate, which may be either crystalline or amorphous.

Specific MFI-type zeolites and processes for their production utilising organic bases are described in US Patent Nos 3 702 886; 3 709 979; 4 205 053; 4 116 099; 4 139 600 and 4 151 189; UK Patent Nos 1 365 318 and 1 567 948 and European patent application publication Nos 2899 and 2900, for example.

Alternatively, the organic base may be replaced by a source of ammonium ions as described in our European patent application publication No. 30811, US Patent No 4 119 556 and UK application publication No 2 018 232A.

The cations normally associated with the zeolithes, for example alkali metal and/or alkaline earth metal ions, organic nitrogen ions and ammonium ions may suitably be exchanged with other cations, for example hydrogen ions, using conventional techniques. Furthermore, it is desirable to calcine the zeolithe, suitably by heating, for example in a stream of air, at a temperature in the range from 400 to 600°C for at least 0.5 hour.

It is to be noted that the separation of the mixtures is dependent on the heat of adsorption of the components in the mixture with respect to the adsorbent used. The heat of adsorption is related not only to the amount of the mixture adsorbed from a given solution and the type of adsorbent used but also on the rate of adsorption. Thus for ethanol and acetic acid, the amounts of adsorption on silicalite are not very different when these compounds are adsorbed from a 10 g/litre solution in water but the heat of adsorption for acetic acid is very much higher which indicates that the rate of adsorption of the acid is much higher than it is for ethanol; the converse applies for the rate of desorption.

The process of the invention is applicable to both the separation of a carboxylic acid from an aqueous solution thereof or for the separation of a carboxylic acid from a mixture thereof with one or more other oxygenated aliphatic compounds. Examples of such other oxygenated aliphatic compounds include other carboxylic acids, alcohols, esters, ketones and aldehydes. The carboxylic acid to be separated and the other oxygenated aliphatic compounds suitably contain 1-8 carbon atoms. The process is particularly suited for separating a carboxylic acid from other carboxylic acids of for concentrating a dilute aqueous solution of the carboxylic acid. Suitable carboxylic acids which may be separated in the aforesaid manner may be selected from formic acid, acetic acid, propionic acid, butyric acid and mixtures thereof. The process of the invention may suitably be applied to relatively dilute aqueous solutions of the carboxylic acid to be separated, for example solutions containing less than 70% by weight of the carboxylic acid, more preferably less than 10% by weight.

It has been found that the adsorption of the carboxylic acids increases markedly with the size of the alkyl group attached to the carboxylic acid group. In a particular aspect therefore the present invention provides a process for separating a $C_x$ carboxylic acid from a mixture thereof with one or more $C_y$ carboxylic acids, where x and y are integers such that y is greather than x, which process comprises contacting the mixture under adsorption conditions with an adsorbent comprising either silicalite or an MFI-type crystalline aminosilicate having a silica to alumina molar ratio greater than 50 : 1. Typically x is 1 and y is an integer from 2-8 preferably from 2 to 4. Thus, a mixture of carboxylic acids which may be separated by the process of the invention is that obtained by the oxidation of paraffinic hydrocarbons at elevated temperature and pressure which typically comprises formic acid, acetic acid, propionic acid and traces of butyric acid or such mixtures obtained during product work-up.

The process may be operated using the mixture to be separated in the liquid phase or the gaseous phase.

The conditions used for adsorbing the mixture on the adsorbent will depend upon (a) the nature of the adsorbent, (b) the components in the mixture to be adsorbed, (c) the concentration of the acid to be separated in the mixture and (d) the type of carboxylic acid to be separated. For instance if a carboxylic acid is to be concentrated from an aqueous solution thereof, such a solution is preferably adsorbed on the adsorbent at a pressure from 1 to 50 bars, a temperature from above the freezing point of the solution to 200°C, most preferably from 5-100°C for an acid concentration from 0.1-99 g/litre, most preferably from 1-50 g/litre.

The separated carboxylic acid may be recovered from the adsorbent by contact with a suitable solvent desorbent which displaces the carboxylic acid from the adsorbent, or by heating the adsorbent. Examples of the solvent desorbents that may be used include water, an alcohol such as ethanol, an ester such as ethyl acetate or other solvents which are more strongly adsorbed on the adsorbent than the acid to be separated. For example acetic acid adsorbed at 25°C is rapidly desorbed with water at 100°C.

Alternatively, desorption can be carried out from vapour phase and the acid recovered from the adsorbent by the application of heat and/or reduced pressure.

The adsorbent may be employed in the form of a dense compact fixed bed which is alternately contacted with the feed mixture and desorbent materials. In the simplest embodiment of the invention the adsorbent is employed in the form of a single static bed in which case the process is only semi-continuous. In an alternative embodiment, two or more static beds are employed, the feed mixture being passed through one or more adsorbent beds at the same time as the desorbent is passed through one or more other beds.

It is preferred to employ the adsorbent in the form of a countercurrent moving bed or in a simulated moving bed countercurrent flow system, the principle of which is described in US Patent No 2 985 589.

The process of the present invention will now be illustrated by reference to the following Examples. In the Examples the following apparatus, method and adsorbents were employed.

*Apparatus and Method*

The apparatus was a commercially available flow calorimeter manufactured by Microscal Limited and fitted with syringe micropumps manufactured under the trade name PERFUSOR IV B. Brown Melsungen AG.

The adsorbents used in the experiments were saturated with triply distilled water. Adsorption experiments were carried out as described in Groszek, A.J., Proc. Roy. Soc. A., 1970, *314*, 473-498. A 1% wt/wt solution of carboxylic acid in water (10 g/litre of water) was percolated through the adsorbents (typically about 100 mg) at the rate of 0.05 ml per minute and the temperature of the solution was maintained at 22°C ± °C. As the volume of the adsorbent bed in the cell is about 0.1 ml, the space velocity of the percolating water is about 30 $h^{-1}$.

The adsorbents were used without any previous degassing treatments i.e. as taken out of bottles where they were stored under air. The rises of temperature taking place in the adsorbent through which solutions were percolated were measured by thermistors inserted in the adsorbent and registered by a chart recorder. Calibration was carried out by passing electrical current through a coil immersed in the centre of the adsorbent beds with the solvents percolating through them.

The estimates of the amount of adsorption were carried out by measuring the time taken for the heat avolution to reach its maximum value, doubling it and assuming that during that time all the solute percolated through the adsorbent is adsorbed. The time interval in question usually corresponds to the major proportion of all the heat evolved during the adsorption process, i.e. the heat of adsorption without the adsorption «tail». The amounts of the solutes adsorbed calculated in this way are of course not very accurate, but give nevertheless some idea of the capacity of the solids to adsorb the solutes under the flow conditions, i.e. most probably non-equilibrium conditions.

*Adsorbents*

*A. MFI-type crystalline aluminosilicate zeolites*

Gels of molar composition:

$XSiO_2 : Al_2O_3 : 5.09\ NaOH : 333.6\ H_2O$ where x = 20 to 50 were prepared and crystallised for 72 hours at 170°C ± 1°C. A typical preparation of a high purity MFI($NH_3$) zeolite using such a formulation is as follows.

3.66 g (44.7 mmols) of sodium aluminate was added to a solution of 2.68 g (67 mmols) of sodium hydroxide in 80.6 g of deionised water and the mixture stirred for 30 min at room temperature during which time complete dissolution occurred (Solution A).

A second solution (B) was prepared meanwhile by adding 1.39 g of aqueous ammonia to 85.8 g of Ludox AS 40 colloidal silica. This solution was always prepared 25 min after the preparation of Solution A so that it was stirred for 5 min only.

As soon as the solution A and B where ready Solution B was added to Solution A with rapid stirring. On mixing, a thick, opaque gel was formed which quickly became mobile with further stirring. After 10 min had elapsed 80 ml of the gel was placed in a clean 100 ml stainless pressure vessel which was immediately sealed and clamped to a rotating shaft in the crystallisation oven. The vessel was slowly rotated while being heated to 170°C, maintained at this temperature for 72 hours and then rapidly cooled to room temperature.

When the pressure vessel was opened the product generally was a white solid suspended in an aqueous medium. The solid was rapidly separated from the mother liquor by filtration and washed with 400 ml of deionised water. The raw zeolite so obtained was dried to constant weight at 120°C.

B. *Silicalite*

60 g of Ludox AS 40, an ammonium stabilised silica sol, was added, with stirring, to 60 g of aqueous tetrapropylammonium hydroxide (5% weight). After stirring for approximately 10 min, the mixture was placed in a 150 ml stainless steel pressure vessel and heated to 170°C for 60 hours under autogenous pressure. On cooling, the vessel was opened and the raw product separated from the mother liquor by filtration. The solid filter cake was then dried to constant weight at 120°C in an oven.

Before use, a sample of the silicalite was calcined in air at 500°C for 60 hours. Powder X-ray diffraction analysis of the calcined sample showed it to be pure crystalline silicalite with no detectable amounts of amorphous material or other zeolithe phases.

*Example 1*

The aforesaid method was used for determining the heat of adsorption, i.e. the heat evolved when water is displaced from the adsorbent, for formic acid using as adsorbent an MFI($NH_3$) zeolite prepared as hereinbefore described and having a Si:Al atomic ratio of 47:1, i.e. a silica to alumina molar ratio of 53.2:1. The amount of formic acid adsorbent was also estimated. The results are given in Table 1.

*Example 2*

The procedure of Example 1 was repeated except that formic acid was replaced by acetic acid. The results are given in Table 1.

*Example 3*

The procedure of Example 1 was repeated except that formic acid was replaced by propionic acid. The results are given in Table 1.

*Comparison Test 1*

The procedure of Example 1 was repeated except that formic acid was replaced by ethanol. The results are given in Table 1.

*Comparison Test 2*

The procedure of Example 1 was repeated except that formic acid was replaced by ammonia. The results are given in Table 1.

*Example 4*

The procedure of Example 1 was repeated except that the MFI($NH_3$) zeolithe was replaced by silicalite and formic acid was replaced by acetic acid. The results are given in Table 2.

*Comparison Test 3*

The procedure of Example 1 was repeated except that the MFI($NH_3$) zeolite having a silica to alumina molar ratio of 53.2:1 was replaced by an MFI($NH_3$) zeolite having a silica to alumina molar ratio of 19.2:1 (Atomic ratio of 17:1) and formic acid was replaced by acetic acid. The results are given in Table 2.

TABLE 1

*Adsorption of carboxylic acids from aqueous solutions using as adsorbent an MFI($NH_3$) zeolite having a silica to alumina ratio of 53.2:1*

| Example | Adsorbate | Heat of adsorption J/g of adsorbent | Amount of adsorption mg/g of adsorbent |
|---|---|---|---|
| 1 | Formic acid | 8.2 | 13.1 |
| 2 | Acetic acid | 17.0 | 45.0 |
| 3 | Propionic acid | 21.2 | 64.0 |
| Comp Test 1 | Ethanol | 13.0 | 53.0 |
| Comp Test 2 | Ammonia | 5.4 | 11.4 |

TABLE 2

*Adsorption of acetic acid on silicalite and MFI($NH_3$) zeolites*

| Example | Adsorbent | Heat of adsorption (J/g of adsorbent) |
|---|---|---|
| 2 | MFI($NH_3$) zeolite (silica : alumina = 53.2) | 17.0 |
| 4 | Silicalite | 27.5 |
| Comp Test 3 | MFI($NH_3$) zeolite (silica : alumina = 19.2) | 8.2 |

*Example 5*

Dilute aqueous solutions of various acids were percolated through ZSM-5 and silicalite adsorbents at 25°C in the concentrations shown in Table 3 below. The table shows that the acids are virtually completely removed from the solutions by silicalite and to a lesser extent by other ZSM-5 adsorbents, with the effluent being virtually pure water (acid concentration less than 0.1 g/litre) until the adsorbent is saturated with the acids. The amount of the acids adsorbed in such percolation experiments are also give in Table 3 below.

TABLE 2

| Acid in 1% wt Solution | Silicate (500:1) mg/g | ZSM (38:1) mg/g |
|---|---|---|
| Formic | 21 | 31 |
| Acetic | 105 | 48 |
| Propionic | 126 | 79 |

These results were obtained at 25°C, a pressure of 6 psi and a flow rate of 15 v/v/h. The silicate used was not the same as those used in the heat of adsorption determinations (different batch).

It was found that the adsorbed acids can be completely recovered from the adsorbents with water at 100°C and the adsorbent used again. For example silicalite was used repeatedly 3 times for the adsorption of acetic acid and the amount of the acid adsorbed from a 10 g/litre solution was each time 110 mg/g ± 5 mg/g.

**Claims**

1. A process for separating a carboxylic acid of 1-8 carbon atoms from a mixture thereof with water and/or one or more other oxygenated aliphatic compounds which process comprises contacting the mixture under adsorption conditions with an adsorbent comprising either silicalite or an MFI-type crystalline aluminosilicate zeolite having a silica to alumina molar ratio greater than 50:1.

2. A process according to claim 1 wherein the carboxylic acid is selected from formic acid, acetic acid, propionic acid, butyric acid and mixtures thereof.

3. A process according to claim 1 wherein the carboxylic acid is separated from an aqueous solution thereof containing less than 70% by weight of the carboxylic acid.

4. A process according to any one of the preceding claims wherein the oxygenated aliphatic compound is selected from an alcohol, an ester, a ketone, an aldehyde and a carboxylic acid other than that being separated.

5. A process according to any one of the preceding claims wherein the process is operated using the mixture to be separated in the liquid phase or the gaseous phase.

6. A process according to any one of the preceding claims wherein the adsorbent is employed in the form of a dense compact fixed bed which is alternately contacted with the feed mixture and desorbent materials.

7. A process according to any one of the preceding claims wherein the adsorbent is employed in the form of a countercurrent moving bed or in a simulated moving bed countercurrent flow system.

**Patentansprüche**

1. Verfahren zur Abtrennung einer Carbonsäure mit 1 bis 8 Kohlenstoff-Atomen aus einer Mischung derselben mit Wasser und/oder einer oder mehreren anderen sauerstoffhaltigen aliphatischen Verbindungen, bei dem die Mischung unter Adsorptionsbedingungen mit einem Adsorptionsmittel aus Silicalit oder einem kristallinen Aluminiumsilicat-Zeolith vom MFI-Typ mit einem Stoffmengenverhältnis («Molverhältnis») Siliciumdioxid zu Aluminiumoxid von mehr als 50:1 in Kontakt gebracht wird.

2. Verfahren nach Anspruch 1, dadurch gekennzeichnet, dass die Carbonsäure aus Ameisensäure, Essigsäure, Propionsäure, Buttersäure und deren Mischungen ausgewählt ist.

3. Verfahren nach Anspruch 1, dadurch gekennzeichnet, dass die Carbonsäure aus einer weniger als 70 Gew.-% der Carbonsäure enthaltenden wässrigen Lösung abgetrennt wird.

4. Verfahren nach irgendeinem der vorhergehenden Ansprüche, dadurch gekennzeichnet, dass die sauerstoffhaltige aliphatische Verbindung aus einem Alkohol, einem Ester, einem Keton, einem Aldehyd und einer anderen als der abzutrennenden Carbonsäure ausgewählt ist.

5. Verfahren nach irgendeinem der vorhergehenden Ansprüche, dadurch gekennzeichnet, dass das Verfahren unter Einsatz der zu trennenden Mischung in der flüssigen Phase oder in der Gasphase betrieben wird.

6. Verfahren nach irgendeinem der vorhergehenden Ansprüche, dadurch gekennzeichnet, dass das Adsorptionsmittel in Form eines dichten, kompakten Festbetts eingesetzt wird, das alternierend mit der Einsatzmischung und desorbierenden Stoffen in Berührung gebracht wird.

7. Verfahren nach irgendeinem der vorhergehenden Ansprüche, dadurch gekennzeichnet, dass das Absorptionsmittel in Form eines Gegenstrom-Bewegtbettes oder in einem Gegenstrom-Fliesssystem mit simuliertem Bewegtbett eingesetzt wird.

**Revendications**

1. Un procédé de séparation d'un acide carboxylique de 1 à 8 atomes de carbone à partir d'un mélange de celui-ci avec de l'eau et/ou un ou plusieurs autres composés aliphatiques oxygénés, procédé qui consiste à mettre le mélange en contact dans des conditions d'adsorption avec un adsorbant comprenant soit de la silicalite soit une zéolite cristalline d'aluminosilicate de type MFI ayant un rapport molaire silice sur alumine plus grande que 50:1.

2. Un procédé selon la revendication 1, dans lequel l'acide carboxylique est choisi à partir de l'acide formique l'acide acétique, l'acide propionique, l'acide butyrique et leurs mélanges.

3. Un procédé selon la revendication 1, dans lequel on sépare l'acide carboxylique d'une solution aqueuse de celui-ci contenant moins de 70% an poids de l'acide carboxylique.

4. Un procédé selon l'une quelconque des revendications précédentes, dans lequel le composé aliphatique oxygéné est choisi à partir d'un alcool, d'un ester, d'une cétone, d'un aldéhyde et d'un acide carboxylique autre que celui que l'un sépare.

5. Un procédé selon l'une quelconque des revendications précédentes, dans lequel on conduit le procédé en utilisant le mélange à séparer en phase liquide ou en phase gazeuse.

6. Un procédé selon l'une quelconque des revendications précédentes, dans lequel on utilise l'adsorbant sous la forme d'une couche fixe compacte dense que l'on met en contact alternativement avec le mélange d'alimentation et des matières désorbantes.

7. Un procédé selon l'une quelconque des revendications précédentes, dans lequel on utilise l'adsorbant sous la forme d'une couche en mouvement à contre-courant ou dans un système simulé d'écoulement à contre-courant en couche mobile.